# EUROPÄISCHE PATENTSCHRIFT

(11) **EP 1 682 705 B1**
(45) Veröffentlichungstag und Bekanntmachung des Hinweises auf die Patenterteilung: **04.05.2011**
(21) Anmeldenummer: 04765067.6
(22) Anmeldetag: 10.09.2004
(51) Int. Cl.: D01H 13/32

(54) **VERFAHREN ZUM ERZIELEN EINER VISUELLEN DARSTELLUNG DER EINHALTUNG VORGEGEBENER EFFEKTE**
METHOD FOR OBTAINING A VISUAL DISPLAY IF PREDETERMINED EFFECTS ARE PRODUCED
PROCEDE DE REALISER UNE REPRESENTATION VISUELLE EN CONFORMITE AVEC LES EFFETS DONNES

(30) Priorität: 16.10.2003 DE 10348741
(43) Veröffentlichungstag der Anmeldung: 26.07.2006
(73) Patentinhaber: Oerlikon Textile GmbH & Co. KG, 42897 Remscheid (DE)
(72) Erfinder: BIERMANN, Iris, 41239 Mönchengladbach (DE); HAASE, Christoph, 41748 Viersen (DE); KREITZEN, Lorenz, 47495 Rheinberg (DE)
(74) Vertreter: Hamann, Arndt
(86) Internationale Anmeldenummer: PCT/EP2004/010141
(87) Internationale Veröffentlichungsnummer: WO 2005/042815

(56) Entgegenhaltungen:
- EP-A1- 0 531 894
- EP-A1- 0 578 975
- US-A- 5 146 550

## Beschreibung

Zur Herstellung von Effektgarn wird die gewünschte Ausbildung des Garnes festgelegt und werden auf der Basis dieser Festlegung die für das Spinnen des Effektgarnes erforderlichen Spinneinstellungen generiert. Dazu werden die Effekte, charakterisiert durch Effektdicke und Effektlänge, und Stege, charakterisiert durch Stegdicke und Steglänge, sowie die Aufeinanderfolge von Stegen und verschiedenen Effekten vorgegeben. Diese Vorgabe wird in einem sogenannten Rapport abgespeichert und ist beispielsweise als virtuelle Garntafel auf einem Bildschirm darstellbar. Eine derartige Darstellung ist bei einer visuellen Überprüfung jedoch nicht ausreichend aussagekräftig, um schnell und eindeutig erkennen zu lassen, ob die vorgegebenen Effekte in der vorgegebenen Verteilung im hergestellten Effektgarn vorliegen.

Die EP 0 531 894 A1 offenbart ein Verfahren zur Ausreinigung von Garnfehlern und zum Erzielen einer visuellen Darstellung der Garnfehler in einer zweidimensionalen Klassiermatrix. Die Klassierung erfolgt durch Messung der Abweichung des Garndurchmessers von Soll-Vorgaben und durch Erfassung der fehlerbehafteten Garnlänge.

Es ist Aufgabe der Erfindung, ein Verfahren zu schaffen, mit dem die Überprüfung, ob die vorgegebenen Effekte im hergestellten Effektgarn wie gewünscht erzeugt worden sind, verbessert werden kann.

Diese Aufgabe wird mit einem Verfahren mit den Merkmalen des Anspruchs 1 gelöst.

Vorteilhafte Ausgestaltungen der Erfindung sind Gegenstand der Unteransprüche.

Das erfindungsgemäße Verfahren stellt eine Information zur Verfügung, in welchen Längen- und Dickenklassen sich die Effekte in welcher Anzahl befinden. Daraus lässt sich sofort und eindeutig ableiten, ob die Effekte in der gewünschten Ausbildung und Anzahl hergestellt worden sind. Das Verfahren ermöglicht sowohl eine visuelle Auswertung als auch eine automatische Auswertung. Eine einfache Auswertung kann durch einen SOLL-IST-Vergleich erfolgen, bei dem die tatsächlich vorliegende und entsprechend in der Klassiermatrix angezeigte Anzahl der Effekte mit der vorgesehenen Anzahl der Effekte in der jeweiligen Klasse verglichen wird. Das Einleiten von Maßnahmen kann von einem bestimmten Grad der Übereinstimmung bzw. der Differenz zwischen der angezeigten Anzahl der Effekte und der vorgesehenen Anzahl der Effekte abhängig gemacht werden. Durch unterschiedliche farbliche Hinterlegung der Klassen, in denen Effekte vorgesehen sind, und der Klassen, in denen keine Effekte vorgesehen sind, läßt sich eine visuelle Überprüfung leichter und schneller durchführen. Aufgrund der Anzeige der Klassiermatrix kann ein Optimieren der Spinneinstellungen ausgelöst werden.

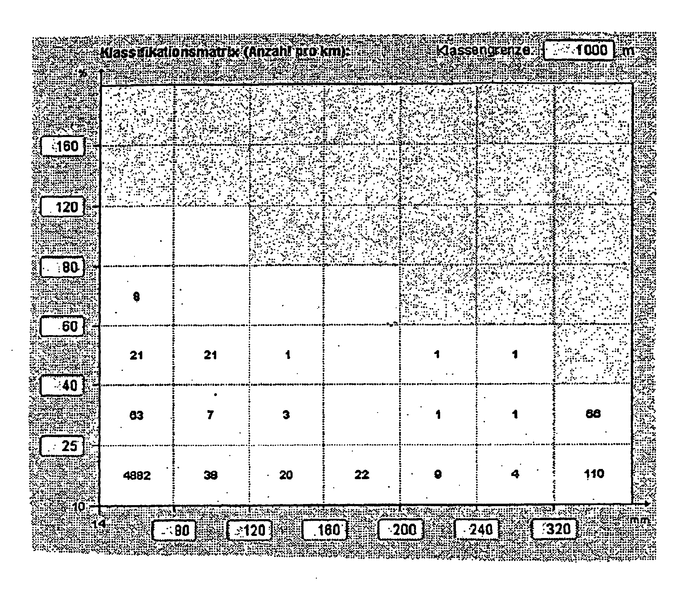

Weitere Einzelheiten der Erfindung sind der Figur entnehmbar. Die Figur zeigt eine Klassiermatrix für Garneffekte, bei der in einer Dimension sieben Durchmesserbereiche von Effekten und in der anderen Dimension sieben Längenbereiche von Effekten dargestellt sind, die insgesamt 49 Klassen bilden. Der erste Durchmesserbereich reicht von einem Durchmesserwert, der 10% über dem Stegdurchmesser liegt bis zu einem Durchmesserwert, der 25% über dem Stegdurchmesser liegt. Der erste Längenbereich reicht von 14 mm bis 80 mm. In der durch diese beiden Bereiche gebildeten Klasse sind auf 1.000 Meter Garnlänge 4.882 Effekte ermittelt worden. Die Verteilung der ermittelten Effekte auf die einzelnen Klassen ist der Darstellung der Figur entnehmbar. Die in den einzelnen Klassen angezeigten Summen beziehen sich auf 1.000 Meter Messlänge. Wird eine geringere Messlänge aufgenommen, werden die Ergebnisse auf 1.000 Meter hochgerechnet. Zusätzlich kann die Summe der Effekte in allen Klassen eines Längenbereiches oder die Summe aller Effekte in den Klassen eines Durchmesserbereiches angezeigt werden. Mit den in der Klassiermatrix angezeigten Summen ist eine schnelle und einfache Überprüfung auf Einhaltung der Effektvorgabe möglich.

Die Grenzen der Längenbereiche und der Dickenbereiche können frei wählbar verändert werden. Die Klassiermatrix der Figur zeigt einen Bereich, in dem die Klassen weiß hinterlegt und einen anderen Bereich - in der Darstellung der Figur rechts oben - der grau hinterlegt ist. Die weiß hinterlegten Klassen repräsentieren Klassen, in denen sich die Effekte befinden sollen. Die grau hinterlegten Klassen repräsentieren die übrigen Bereiche. Umfaßt beispielsweise die gewünschte Effektausbildung nur Effekte, bei denen die Effektdicke jeweils um wenigstens 25% über der Stegdicke liegt, sind in der entsprechenden, hier nicht dargestellten Klassiermatrix die sieben Klassen des Dickenbereiches von 10% bis 25%, die in der unteren Reihe der Darstellung der Figur weiß hinterlegt sind, nun nicht mehr weiß, sondern grau hinterlegt. Wenn in diesen grau hinterlegten Klassen Effekte angezeigt werden, ist offenkundig, daß das Effektgarn nicht vorgegebene Effekte enthält. Das Anzeigen von Effekten in den grau hinterlegten Klassen kann eine Optimierung der Spinneinstellungen auslösen mit dem Ziel, diese unerwünschten Effekte zukünftig bei der Herstellung des Effektgarnes zu eliminieren.

Dir Darstellung der Klassiermatrix kann auf einem Bildschirm oder durch Ausdruck erfolgen.

Die Erfindung ist nicht auf die dargestellte Klassiermatrix beschränkt. Im Rahmen der Erfindung sind weitere Ausbildungen der Klassiermatrix möglich.

## Patentansprüche

1. Verfahren zur Herstellung von Effektgarn, wobei zur Einhaltung vorgegebener Effekte Abweichungen des Garndurchmessers von Soll-Vorgaben gemessen werden,
wobei bei Abweichungen des Garndurchmessers von Soll-Vorgaben sowohl die Größe der Abweichung als auch ihre Ausdehnung in Längsrichtung des Effektgarns gemessen wird, wobei die Effektbereiche des Effektgarns in einer zweidimensionalen Klassiermatrix erfasst werden und den Klassen der Klassiermatrix zugeordnet und dort summiert werden, wobei als Effektbereiche nur die Bereiche des Effektgarnes gelten, deren Durchmesser mindestens einen vorbestimmten Betrag über einem vorgegebenen Stegdurchmesser liegt und deren Länge eine vorbestimmte Mindestlänge überschreitet,
wobei die Klassiermatrix in einer Dimension in Längenbereiche und in der anderen Dimension in Durchmesserbereiche unterteilt ist und durch Kombination eines Längenbereiches mit einem Durchmesserbereich jeweils eine Klasse bildet,
wobei die in der Klassiermatrix angezeigten Anzahl mit der vorgesehenen Anzahl der Effekte in der jeweiligen Klasse verglichen wird,
und wobei in Abhängigkeit von der Differenz die Spinneinstellungen optimiert werden.

2. Verfahren nach Anspruch 1, **dadurch gekennzeichnet, dass** als vorbestimmter Betrag für den Durchmesser ein Betrag gewählt wird, der mindestens 10% über dem Stegdurchmesser liegt.

3. Verfahren nach Anspruch 1 oder 2, **dadurch gekennzeichnet, dass** als vorbestimmte Mindestlänge der angezeigten Effekte eine Länge von 14 mm gewählt wird.

4. Verfahren nach einem der Ansprüche 1 bis 3, **dadurch gekennzeichnet, dass** die Effekte auf jeweils 1.000 Meter Garnlänge erfasst werden.

5. Verfahren nach einem der Ansprüche 1 bis 4, **dadurch gekennzeichnet, dass** die Grenzen der Klassen veränderbar sowie frei wählbar sind.

6. Verfahren nach einem der Ansprüche 1 bis 5, **dadurch gekennzeichnet, dass** jeweils sieben Längenbereiche und Durchmesserbereiche ausgewählt werden.

## Claims

1. Method for producing fancy yarn, wherein to maintain predefined effects any deviations in the yarn diameter from reference specifications are measured, wherein in the case of differences in the yarn diameter from reference specifications both the size of the difference and also its extent in the longitudinal direction of the fancy yarn are measured, wherein the fancy effect sections of the fancy yarn are recorded in a two-dimensional classifying matrix, assigned to the classes of the classifying matrix and totalled there, wherein only those sections of fancy yarn count as fancy sections, which have a diameter that is at least a predetermined amount above a prespecified web diameter and which have a length which exceeds a predetermined minimum length, wherein the classifying matrix is divided in one dimension into longitudinal sections and in the other dimension into diameter sections and forms a class in each case by combining a longitudinal section with a diameter section,
wherein the number displayed in the classifying matrix is compared with the intended number of effects in the respective class, and wherein the spinning settings are optimised as a function of the difference.

2. Method according to claim 1, **characterised in that** as the prespecified amount for the diameter an amount is selected which is at least 10% above the web diameter.

3. Method according to claim 1 or 2, **characterised in that** a length of 14 mm is selected as the predetermined minimum length of displayed effects.

4. Method according to any one of claims 1 to 3, **characterised in that** the effects are recorded over 1,000 metres of yarn length in each case.

5. Method according to one of claims 1 to 4, **characterised in that** the limits of the classes can be changed and can be selected freely.

6. Method according to one of claims 1 to 5, **characterised in that** seven longitudinal sections and diameter sections are selected in each case.

## Revendications

1. Procédé de production d'un fil fantaisie impliquant, en vue d'entretenir des effets de fantaisie préétablis, une mesure d'écarts du diamètre du fil par rapport à des données de consigne préétablies, sachant que, lorsque ledit diamètre du fil accuse des écarts vis-à-vis de données de consigne préétablies, la mesure porte tant sur la grandeur de l'écart, que sur son étendue dans la direction longitudinale dudit fil fantaisie ; sachant que les zones de fantaisie du fil fantaisie sont saisies dans une matrice de classification bidimensionnelle, puis affectées aux classes de ladite matrice de classification dans laquelle elles sont additionnées, seules étant considérées, comme des zones de fantaisie, les zones dudit fil fantaisie dont le diamètre excède un diamètre d'âme préétabli, d'au moins une valeur prédéterminée, et dont la longueur excède une longueur minimale prédéterminée,
ladite matrice de classification étant scindée en des plages de longueurs, dans une dimension, et en des plages de diamètres dans l'autre dimension, et formant respectivement une classe par combinaison d'une plage de longueurs et d'une plage de diamètres,
le nombre, affiché dans ladite matrice de classification, étant comparé au nombre prévu d'effets de fantaisie dans la classe considérée,
et les réglages du filage étant optimalisés en fonction de la différence.

2. Procédé selon la revendication 1, **caractérisé par le fait qu'**une valeur, excédant d'au moins 10 % le diamètre de l'âme, est choisie en tant que valeur prédéterminée s'appliquant au diamètre.

3. Procédé selon la revendication 1 ou 2, **caractérisé par le fait qu'**une longueur de 14 mm est choisie en tant que longueur minimale prédéterminée des effets de fantaisie affichés.

4. Procédé selon l'une des revendications 1 à 3, **caractérisé par le fait que** les effets de fantaisie sont détectés, à chaque fois, sur 1 000 mètres de longueur de fil.

5. Procédé selon l'une des revendications 1 à 4, **caractérisé par le fait que** les limites des classes peuvent être aussi bien modifiées, que choisies librement.

6. Procédé selon l'une des revendications 1 à 5, **caractérisé par le fait que** sept plages de longueurs, et plages de diamètres, sont sélectionnées à chaque fois.
